Europäisches Patentamt

European Patent Office

Office européen des brevets

(19)

(11) **EP 0 873 716 B1**

(12) **FASCICULE DE BREVET EUROPEEN**

(45) Date de publication et mention
de la délivrance du brevet:
**12.01.2005 Bulletin 2005/02**

(51) Int Cl.⁷: **A61B 8/06**

(21) Numéro de dépôt: **97204011.7**

(22) Date de dépôt: **18.12.1997**

(54) **Système d'échographie ultrasonore pour l'examen des artères**

Ultraschall-Echographiesystem zur Untersuchung von Arterien

Ultrasonic echography system for examination of arteries

(84) Etats contractants désignés:
**DE FR NL**

(30) Priorité: **31.12.1996 FR 9616265**

(43) Date de publication de la demande:
**28.10.1998 Bulletin 1998/44**

(73) Titulaire: **Koninklijke Philips Electronics N.V.**
**5621 BA Eindhoven (NL)**

(72) Inventeurs:
• **Bonnefous, Odile**
**75008 Paris (FR)**

• **Gendreu, Philippe**
**75008 Paris (FR)**

(74) Mandataire: **Charpail, François et al**
**Société Civile S.P.I.D.**
**156, Boulevard Haussmann**
**75008 Paris (FR)**

(56) Documents cités:
**EP-A- 0 035 213**          **US-A- 3 888 238**

## Description

L'invention concerne un système d'échographie ultrasonore pour l'examen des artères comportant une sonde au moins un transducteur ultrasonore reliée à un échographe constitué d'un étage d'émission d'un faisceau ultrasonore, d'un étage de réception et d'un étage de traitement des signaux ultrasonores renvoyés vers la sonde ainsi qu'un dispositif d'affichage d'imagerie ultrasonore de l'artère à examiner.

L'invention trouve son application dans l'industrie de l'imagerie médicale échographique et de la mesure de paramètres physiologiques.

L'échographie ultrasonore est devenue progressivement un moyen efficace de visualisation des tissus, des artères notamment, et il devient possible de nos jours de mesurer avec une assez bonne précision des paramètres physiologiques. Pour ce qui est de l'examen d'une artère, qu'il s'agisse de sa visualisation en coupe ou de la mesure instantanée de la vitesse du flux sanguin qui parcourt cette artère, le maniement de la sonde échographique par le praticien reste toujours délicat et peut se révéler être une source de mauvaise interprétation de l'image obtenue ou une source d'erreur lors d'une analyse quantitative.

Une difficulté rencontrée en cas de mesure quantitative est l'obtention d'un positionnement correct de la sonde par rapport à l'axe du vaisseau (de l'artère). Par positionnement correct on entend, le plus souvent, faire en sorte que le plan de coupe engendré par le faisceau ultrasonore, dans lequel se trouve l'image visualisée, passe par l'axe de l'artère.

A ce jour certains critères d'acquisition des données en vue d'une mesure correcte des vitesses sanguines ont pu être définis pour l'utilisation d'une sonde classique, qu'elle soit linéaire ou courbe. Il s'agit par exemple, pour le praticien, d'effectuer 5 fois la mesure, pour un tronçon d'artère considéré, et de ne retenir parmi ces 5, que les 3 qui se rapprochent le plus entre elles. Cependant, cette technique est longue et assez difficile à mettre en oeuvre et certains échographistes souhaitent avoir une procédure plus courte tout en étant plus fiable, ce qui constitue un problème technique à résoudre. Etant donnés les progrès faits dans les techniques échographiques en vitesse et en précision de calcul, il devient maintenant souhaitable de pouvoir obtenir une visualisation d'artère plus achevée, pour faciliter un examen vasculaire et permettre l'acquisition de données vasculaires plus fiables.

Le brevet US 3,888,238 décrit un système d'échographie ultrasonore pour l'examen des artères selon le préambule de la revendication 1. Ce système comporte notamment une sonde comprenant un transducteur ultrasonore et un échographe relié à la sonde. La sonde se compose d'un transducteur constitué par un assemblage de trois réseaux de transducteurs élémentaires solidaires en forme de H, un premier réseau C de transducteurs étant apte à explorer l'artère dans un sens axial et un deuxième réseau A et un troisième réseau B de transducteurs latéraux, dont les plans d'explorations sont parallèles entre eux et sont perpendiculaires au plan d'exploration du premier transducteur.

Un but de l'invention est de réaliser un système d'échographie ultrasonore qui permette d'obtenir en temps réel des images en coupe d'artère selon lesquelles le plan de coupe de l'artère passe par l'axe de cette dernière. L'invention est définie dans la revendication 1.

Un autre but est de réaliser un système d'échographie ultrasonore qui permette d'obtenir des mesures de vitesses sanguines avec une fiabilité améliorée.

Encore un autre but est de réaliser une sonde à transducteurs ultrasonores qui permette, par guidage d'images complémentaires sur un dispositif d'affichage, d'ajuster l'image en coupe d'une artère pour qu'elle passe par l'axe de cette dernière.

Ces buts sont atteints et les inconvénients de l'art antérieur sont atténués grâce au fait que le système d'échographie ultrasonore défini en préambule est remarquable en ce que ladite sonde se compose d'un assemblage de trois transducteurs solidaires, un premier transducteur médian étant conçu pour explorer ladite artère dans un sens axial et un deuxième et un troisième transducteurs latéraux, accolés symétriquement audit premier transducteur et orientés de façon à explorer l'artère en substance transversalement, selon des plans d'exploration perpendiculaires au plan d'exploration dudit premier transducteur, et que ledit échographe comporte des moyens des moyens de mutiplexage pour exciter successivement lesdits premier, deuxième et troisième transducteurs.

On notera que sont déjà connues des sondes à deux plans d'exploration pour l'imagerie cardiaque telles celles vendues par la société américaine Hewlett Packard pour examens trans-oesophagiens.

Le dispositif d'affichage du système est conçu de façon à représenter trois images échographiques obtenues à partir des trois transducteurs solidaires, respectivement. L'image principale, qui peut être suivie ultérieurement d'une phase de mesure dans une partie d'intérêt d'une artère qu'elle met en évidence, est fournie par le transducteur médian. Les deux images latérales en direction perpendiculaire à l'image principale et parallèles entre elles servent quant à elles à guider le praticien dans son exploration de l'artère en question et lui permettent d'obtenir la section longitudinale de surface maximale lorsqu'il le désire, ce qui est généralement le cas. Pour cela il doit, dans un premier temps, faire apparaître selon une forme ellipsoïdale une section sensiblement transversale de l'artère sur chaque image latérale, ce qui assure l'acquisition de l'image principale selon toute la longueur du premier transducteur médian puis, en continuant de manipuler la sonde, centrer ces deux images en section transversale, de façon que l'axe de symétrie qui partage, dans le sens du tir chaque plan d'image transversale, partage aussi symétriquement chaque section transvesale. Ces axes de symétrie, parallèles, qu'il est avantageux de matérialiser sur les plans d'images transversales, sous forme d'un trait sur l'écran, sont les intersections du plan d'image médian avec chaque plan d'image transversale. Cette dernière opération constitue une sorte de mise au point de l'image souhaitée : elle assure que l'image principale fournie par le transducteur médian contient l'axe de l'artère. Cette position relative de la sonde par rapport à l'artère est la plus favorable pour effectuer un tir selon une droite de l'image principale, tir en mode Doppler pulsé destiné à fournir notamment les composantes des vitesses sanguines selon la direction du tir, le tir étant répété, de préférence, pendant la durée d'au moins une pulsation cardiaque.

Selon l'invention, les moyens de séquencement que comporte l'échographe adapté à la sonde à trois plans d'exploration décrite ci-dessus, sont constitués par deux multiplexeurs disposés en cascade entre ledit étage d'émission des signaux ultrasonores et ladite sonde, un premier multiplexeur muni de premiers moyens de commande pour faire l'alternance entre d'une part les signaux destinés audit premier transducteur et d'autre part les signaux destinés auxdits deuxième et troisième transducteurs, et un deuxième multiplexeur disposé en aval dudit premier multiplexeur et muni de deuxièmes moyens de commande pour faire l'alternance entre les signaux destinés auxdits deuxième et troisième transducteurs respectivement.

La séquence de fonctionnement en émission-réception des trois transducteurs se répète cycliquement, à une fréquence suffisamment élevée pour pouvoir visualiser, sur le dispositif . d'affichage d'imagerie, en temps réel, l'image fournie par chacun des trois transducteurs de la sonde échographique, simultanément.

La description qui suit en regard des dessins annexés, le tout donné à titre d'exemple non limitatif, fera bien comprendre comment l'invention peut être réalisée.

La figure 1 est une vue frontale de la sonde à trois transducteurs selon l'invention.

La figure 2 montre en perspective les trois plans d'exploration de la sonde selon l'invention, correctement orientés par rapport à un segment d'artère.

La figure 3 permet d'expliciter comment les images fournies par les transducteurs latéraux permettent de guider la sonde dans la position correcte de la figure 2.

La figure 4 montre le dispositif d'affichage d'imagerie du système d'échographie ultrasonore selon l'invention.

La figure 5 est le schéma synoptique d'un mode de réalisation du système d'échographie ultrasonore selon l'invention.

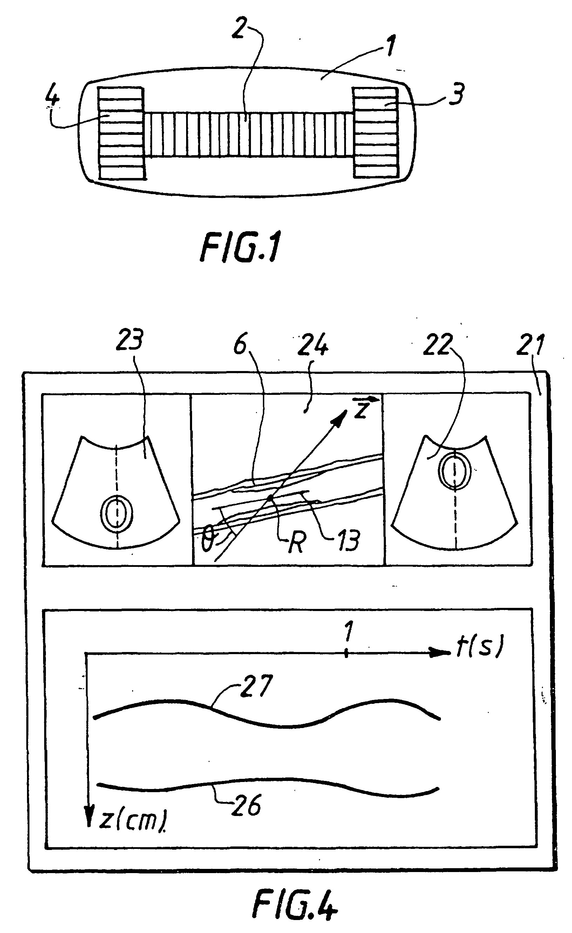

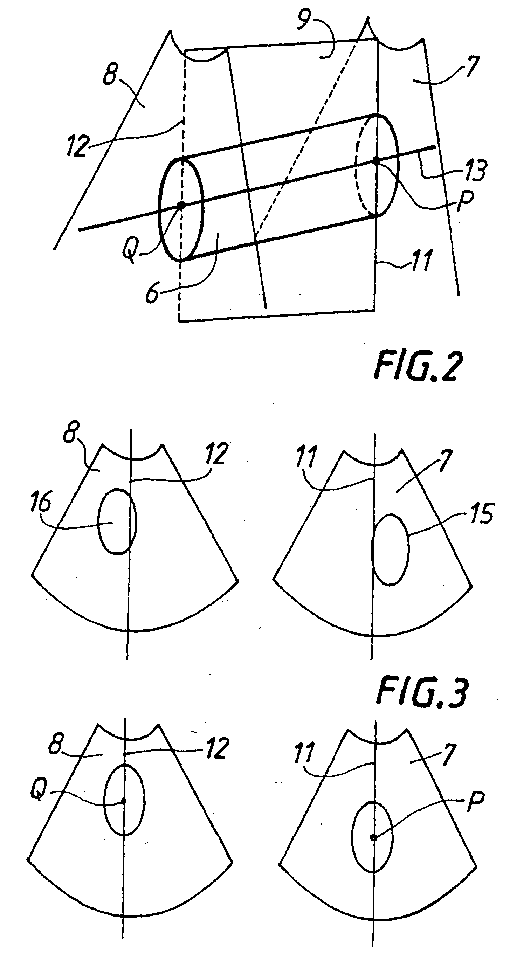

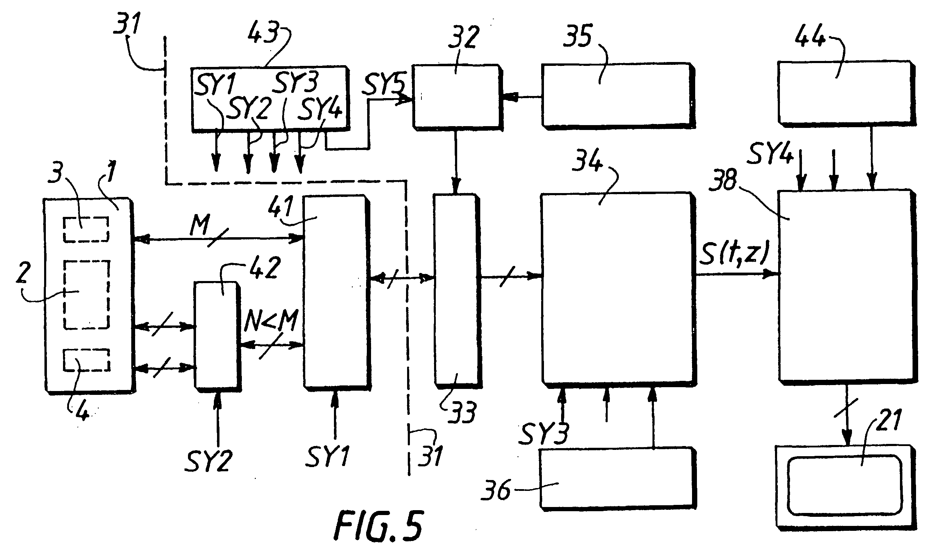

La sonde échographique 1 représentée sur la figure 1 sensiblement à l'échelle 2 se compose de trois transducteurs, le principal étant un transducteur médian 2, conçu pour explorer, selon un plan de coupe frontal, dit médian, perpendiculaire au plan de la figure, un vaisseau sanguin, généralement une artère. Le plus souvent, l'échographiste souhaite obtenir une coupe longitudinale de l'artère et s'il veut pouvoir mesurer les vitesses sanguines les plus élevées à l'intérieur de cette dernière, il est nécessaire que le plan de coupe frontal médian précité contienne l'axe du segment d'artère exploré, de longueur sensiblement égale à celle du transducteur 2. Jusqu'à présent, ce positionnement particulier de la sonde s'obtient de façon empirique et avec une précision faible qui n'est plus en accord avec les performances des échographes modernes. Afin d'obtenir le positionnement précité, il est prévu, selon l'invention, d'adjoindre au transducteur 2, deux transducteurs latéraux, 3 et 4, placés à chaque extrémité, de façon que l'ensemble des trois transducteurs 2, 3 et 4 possède un plan de symétrie frontal (plan 9, sur la figure 2). Les transducteurs 3 et 4 sont conçus pour explorer les tissus selon des plans de coupe frontaux, qui sont parallèles entre eux et perpendiculaires au plan de coupe frontal médian du transducteur 2. Le but recherché, au moyen de la sonde de la figure 1, est d'obtenir sur l'écran d'un moniteur, une représentation en coupe d'une artère qui se décompose en trois images, comme représenté en haut de la figure 4, décrite plus loin. Les transducteurs 2, 3 et 4 se décomposent chacun en transducteurs élémentaires, (représentés sous la forme de petits rectangles) commandés électroniquement avec des lois de retard appropriées pour la focalisation en émission et en réception, qui permettent d'obtenir de façon bien connue un nombre prédéterminé de points d'image par ligne de tir et sensiblement le même nombre de lignes de tir que le nombre de transducteurs élémentaires du transducteur. La matrice de points ainsi obtenus constitue, après traitement analogique, puis le plus souvent numérique, du signal, l'image affichée sur l'écran du moniteur.

Les transducteurs 2, 3 et 4 peuvent être du type transducteur linéaire, tel le LA7530E de la société Philips, ou du type transducteur courbe, tel le CA6414 aussi de Philips, ce dernier permettant d'obtenir des images sectorielles. De préférence, le transducteur médian 2 est du type linéaire et les transducteurs latéraux 3 et 4, du type courbe.

La figure 2 représente schématiquement un segment d'artère 6, encadré par deux plans latéraux 7 et 8 et traversé par un plan médian 9, qui sont les plans d'exploration engendrés par les transducteurs 3, 4 et 2 de la figure 1, respectivement. Le plan 9 coupe le secteur sectoriel du plan 7 en son milieu selon la droite 11 et celui du plan 8 en son milieu selon la droite 12. Sur la figure 2, on se trouve dans la position optimale recherchée pour la sonde, selon laquelle l'axe 13 de l'artère 6 coupe les droites 11 et 12 en P et Q, c'est-à-dire où le plan 9 contient l'axe 13. La recherche de la position optimale par l'échographiste au moyen de la sonde se fait comme suit :

La recherche d'une portion d'artère commence de façon connue par l'obtention d'une coupe de forme ellipsoïdale de cette artère, dans le plan 9 engendré par le transducteur médian 2 ; puis on fait en sorte que la trace de cette coupe occupe toute la largeur utile du plan 9 ; à ce stade, des coupes sensiblement transversales de l'artère apparaissent dans les plans 7 et 8, comme représenté en 15 et 16 en haut de la figure 3. La fin de la phase de positionnement optimal consiste alors à faire bouger très légèrement la sonde de façon à centrer les deux traces 15 et 16 sur les droites 11 et 12 comme représenté au bas de la figure 3. La sonde étant maintenue immobile dans cette position correcte, il devient alors possible d'effectuer des mesures de vitesses du sang, au moyen d'un tir en mode Doppler pulsé, y compris les vitesses des cibles échographiques (globules) qui se trouvent au centre de l'artère (les plus élevées), ce qui permet, après intégration, de calculer avec précision le flux sanguin en fonction du temps, par exemple pendant la durée d'au moins un cycle cardiaque.

La visualisation des images dans les plans 7, 8 et 9 peut être effectuée comme représenté à la partie haute de la figure 4 qui montre l'écran 21 d'un moniteur utilisé en tant que dispositif d'affichage d'imagerie du système d'échographie ultrasonore selon l'invention. Sur cet écran, on a représenté les images latérales 22 et 23 issues des plans de coupe 7 et 8, disposées de part et d'autre de l'image médiane 24 du plan de coupe 9. On notera que ces deux images latérales pourraient aussi être représentées, séparées, l'une au-dessus de l'autre.

La figure 4 affiche la configuration d'exploration de la figure 2. Sur l'image 24 figurent aussi l'axe 13 de l'artère et la direction (l'axe) $\vec{z}$ selon laquelle seront effectués de façon connue après positionnement optimal de la sonde, des tirs répétitifs d'impulsions ultrasonores pour les mesures de vitesses sanguines. Grâce à l'invention les axes 13 et $\vec{z}$ se coupent, au point R, ce qui est le but recherché.

Les vitesses instantanées mesurées sont les composantes sur l'axe $\vec{z}$ des vitesses réelles cherchées. Une correction multiplicative en $1/\cos\theta$ permet de passer des vitesses mesurées aux vitesses réelles, $\theta$ étant l'angle entre les axes 13 et $\vec{z}$. Les vitesses réelles peuvent être stockées sous forme numérique dans une mémoire (non représentée). Elles peuvent aussi être visualisées selon tout mode connu, par exemple en mode M comme représenté au bas de l'écran de la figure 4 : les coordonnées sont le temps, en abscisses et la profondeur z, grandeur scalaire mesurée perpendiculairement à l'axe 13, en ordonnées. Sur la figure 4 sont représentées des traces 26 et 27 qui représentent les parois de l'artère 6 pour une durée de préférence supérieure à celle d'un cycle cardiaque. Entre les traces 26 et 27, les vitesses sanguines, sur le diamètre de l'artère sont visualisées par des couleurs selon un code prédéterminé généralement affiché sur l'écran (de façon non représentée à la figure 4).

Pour obtenir les résultats décrits ci-dessus au moyen de la sonde à trois transducteurs (et trois plans de coupe) de la figure 1, il est nécessaire d'adapter l'échographe auquel est raccordée cette sonde particulière. Plus précisément, cet échographe doit comporter des moyens de séquencement pour exciter en séquence, de façon cyclique, les transducteurs 2, 3 et 4, comme décrit ci-dessous en référence à la figure 5. Pour la commande de la sonde 1 il se pose le problème technique que les trois transducteurs 2, 3 et 4 ne peuvent pas être excités en même temps comme on pourrait envisager qu'ils le soient, chacun par un échographe indépendant ; en effet, ces trois transducteurs sont trop rapprochés et il se créerait, à l'émission et la réception des signaux ultrasonores émis selon trois nappes jointives, des interférences telles que les signaux reçus, entachés de bruit, seraient inexploitables. La solution retenue pour pallier cet inconvénient, consiste à activer, au moyen d'un seul échographe, les trois transducteurs en séquence, au moyen de multiplexeurs, de façon qu'à tout instant seul l'un des trois transducteurs soit activé lorsque le système est en fonctionnement.

Sur la figure 5, la partie située à droite d'un trait interrompu 31 est la structure classique d'un échographe, qu'il soit du type fréquentiel, dit Doppler, ou du type à corrélation temporelle :

Un étage d'émission 32 comprend un séquenceur composé d'un oscillateur et d'un diviseur de fréquence qui commande à la fréquence de récurrence choisie un générateur dont les signaux électriques d'excitation sont envoyés vers la sonde qui les convertit en trains périodiques de signaux impulsionnels ultrasonores. Un séparateur 33 des étages d'émission 32 et de réception et de traitement 34 (dit Frond End, en anglais) est inséré entre la sonde 1 et lesdits étages 32, 34, et évite l'aveuglement des circuits de réception par les signaux d'émission. Pour une configuration en émission donnée, fournie par une mémoire 35 au circuit 32, par exemple pour l'émission du transducteur 2, les M signaux reçus par les M transducteurs élémentaires du transducteur 2 sont fournis au circuit 34 appelé aussi dispositif de formation de voies en réception. Une commande de balayage électronique (non représentée) des transducteurs élémentaires du transducteur 2, respectivement 3, 4, permet le choix de telle ou telle ligne de tir sans déplacement du transducteur, ces lignes étant disposées dans un même plan, soit parallèles entre elles, pour le transducteur 2, soit en éventail, pour les transducteurs 3 et 4. Quant au dispositif de formation de voies 34 il permet, par des configurations de retards particulières qu'il reçoit d'une mémoire de configuration en réception 36 et qui sont imposées aux signaux reçus du séparateur 33, d'obtenir, par focalisations sucessives, autant de points que prévu sur la ligne de tir choisie en 2, 3 ou 4, du point le plus proche vers le plus éloigné. Le circuit de formation de voies 34 fournit au circuits de traitement de signal situé en aval 38, dit back end en anglais, un signal de sortie S(t,z).

Pour un échographe muni d'une sonde à un seul transducteur, un seul plan est balayé cycliquement à une fréquence, comprise entre quelques hertz et quelques dizaines de hertz, suffisamment élevée pour permettre une visualisation confortable, en 21, même lorsque la sonde se déplace. Selon l'invention, dès que le balayage est terminé par l'un des transducteurs 2, 3, 4, pour la fourniture d'une première image, commence le balayage par un deuxième transducteur, ce balayage étant immédiatement suivi du balayage par le troisième transducteur, et ainsi de suite, selon un cycle de durée prédéterminée. Les durées de balayage sont égales à T2, T3 et T4, pour les transducteurs 2, 3 et 4 respectivement, de façon que : T2 + T3 + T4 = T, T étant la durée du cycle précité, de façon telle que la fréquence de défilement des trois images sur l'écran 21 (figure 4), soit égale à 1/T. Ce fonctionnement particulier est obtenu au moyen de deux multiplexeurs 41 et 42, synchronisés de façon adéquate à partir d'un générateur d'horloge 43.

Le multiplexeur 41 est commandé (SY1) de façon à émettre et recevoir pendant la durée T2 vers le transducteur 2 et pendant la durée T3 + T4 qui suit vers le multiplexeur 42. Le multiplexeur 42 est commandé (SY2) de façon à émettre et recevoir pendant la durée T3 vers le transducteur 3 et pendant la durée T4 vers le transducteur 4. A chaque période T, les circuits 32 et 34 sont configurés en conséquence, en émission et en réception pour s'adapter à chacune des trois sondes, au moyen des synchronisations SY3 et SY5 qui sont de préférence les mêmes, à savoir une première configuration pendant la durée T2 pour le fonctionnement du transducteur 2 et une deuxième configuration, pendant la durée T3 + T4 pour le fonctionnement des transducteurs 3, puis 4.

Le signal S(t,z) ainsi obtenu en séquence ternaire est ensuite traité en 38, où est effectuée une conversion de balayage numérique, de façon à fournir sur l'écran 21 les images décrites ci-dessus en référence à la figure 4. A cet effet il reçoit un signal issu d'une mémoire de configuration pour visualisation 44 et un signal de synchronisation SY4 qui permet l'affichage d'une nouvelle image 22 pendant la durée T3, suivi de l'affichage d'une nouvelle image 23 pendant la durée T4, suivi de l'affichage d'une nouvelle image 24 pendant la durée T2 et ainsi de suite, cycliquement. De préférence, pour éviter un effet de papillotement sur l'écran, la durée de chaque nouvelle image est maintenue pendant une durée égale à T.

On notera que sont connus des échographes munis de plusieurs sondes spécialisées pour différentes applications et que, lorsque l'une de ces sondes est choisie par l'échographiste, l'échographe se configure automatiquement, en 32, 34 et 38, pour s'adapter à cette sonde particulière, raison pour laquelle il n'est pas nécessaire de décrire plus en détail ici comment peut s'effectuer cette adaptation de l'échographe à la sonde qui est activée ; autrement dit, il est connu qu'un front-end échographique se configure automatiquement pour une géométrie donnée de sonde et pour un mode de fonctionnement de balayage donné. Pour la mise en oeuvre de l'invention, il est possible d'utiliser l'échographe SD800 de la société Philips, adapté comme décrit ci-dessus.

**Revendications**

**1.** Système d'échographie ultrasonore pour l'examen des artères comportant une sonde (1) à au moins un transducteur ultrasonore (2) et un échographe relié à la sonde et constitué d'un étage d'émission d'un faisceau ultrasonore (32), d'un étage de réception (34) et d'un étage de traitement des signaux ultrasonores renvoyés vers la sonde ainsi qu'un dispositif d'affichage (21) d'imagerie ultrasonore de l'artère à examiner, ladite sonde se composant d'un assemblage de trois transducteurs solidaires (2, 3, 4), un premier transducteur médian (2) étant conçu pour explorer ladite artère dans un sens axial et un deuxième et un troisième (3, 4) transducteurs latéraux, accolés symétriquement audit premier transducteur (2) et orientés de façon à explorer l'artère en substance transversalement, selon des plans d'exploration (7, 8) perpendiculaires au plan d'exploration (9) dudit premier transducteur, ledit échographe comportant des moyens de séquencement (41, 42) pour exciter en séquence, de façon cyclique, lesdits premier (2), deuxième et troisième (3, 4) transducteurs, **caractérisé en ce que** lesdits moyens de séquencement sont constitués par deux multiplexeurs (41, 42) disposés en cascade entre ledit étage d'émission des signaux ultrasonores et ladite sonde (1), un premier multiplexeur (41) muni de premiers moyens de commande (SY1) pour faire l'alternance entre d'une part les signaux destinés audit premier transducteur (2) et d'autre part les signaux destinés auxdits deuxième et troisième transducteurs (3, 4), et un deuxième multiplexeur (42) disposé en aval dudit premier multiplexeur et muni de deuxièmes moyens de commande (SY2) pour faire l'alternance entre les signaux destinés auxdits deuxième (3) et troisième transducteurs (4) respectivement.

**2.** Système d'échographie ultrasonore selon la revendication 1, **caractérisé en ce que** ledit étage de traitement de signaux comporte des moyens de traitement pour afficher séparément et en temps réel sur ledit dispositif d'affichage (21) les trois images ultrasonores obtenues à partir desdits premier (2), deuxième et troisième transducteurs (3, 4).

**Claims**

**1.** An ultrasonic echography system for the examination of arteries, including a probe (1) with at least one ultrasonic transducer (2) and an echograph which is connected to the probe and is formed by a stage for the transmission of an ultrasonic beam (32), a stage for the reception (34) and a stage for the processing of ultrasonic signals returned to the probe, and also a device for displaying (21) the ultrasonic image of the artery to be examined, said probe consisting of an assembly of three integral transducers (2, 3, 4), a first, central, transducer (2) being designed so as to scan said artery in an axial direction whereas a second (3) and a third (4), lateral transducer symmetrically attached to said first transducer (2) and oriented so as to scan the artery substantially transversely in scanning planes (7, 8) extending perpendicularly to the scanning plane (9) of said first transducer, said echograph including sequencing means (41, 42) for sequentially and cyclically exciting said first (2), second (3) and third (4) transducer, **characterized in that** the first sequencing means are formed by two multiplexers (41, 42) connected in a cascade arrangementbetween said stage for the transmission of ultrasonic signals and said probe (1), a first multiplexer (41) provided with first control means (SY1) for alternation between the signals intended for said first transducer (2), on the one hand, and the signals intended for said second (3) and third (4) transducer, on the other, and a second multiplexer (42) arranged downstream from said first multiplexer and provided with second control means (SY2) for alternation between the signals intended for said second (3) and third (4) transducer, respectively.

**2.** An ultrasonic echography system as claimed in claim 1, **characterized in that** said signal processing stage includes processing means for real-time, separate display of the three ultrasonic images obtained from said first (2), second (3) and third (4) transducer on said display device (21).

**Patentansprüche**

**1.** Ultraschall-Echographiesystem für die Untersuchung von Arterien mit einer Sonde (1) mit mindestens einem Ultraschall-Transducer (2) und einem mit der Sonde verbundenen Echographen, gebildet aus einer Ultraschallstrahl-Sendestufe (32), einer Empfangsstufe (34), einer Verarbeitungsstufe der zur Sonde reflektierten Ultraschallsignale sowie einer Anzeigevorrichtung (21) für Ultraschallbilder der zu untersuchenden Arterie, wobei sich die besagte Sonde aus drei fest miteinander verbundenen Transducern (2, 3, 4) zusammensetzt, ein erster mittlerer Transducer (2), der konzipiert ist, um die besagte Arterie in einer axialen Richtung zu analysieren, und ein zweiter und dritter seitlicher Transducer (3, 4), symmetrisch an den besagten ersten Transducer (2) angebaut und in einer Weise angeordnet, um die Arterie im Wesentlichen transversal gemäß zur Analysefläche (9) des besagten ersten Transducers senkrechten Analyseflächen (7, 8) zu analysieren, wobei der besagte Echograph Ablaufsteuerungsmittel (41, 42) enthält, um den ersten (2), zweiten und dritten (3, 4) Transducer sequentiell und zyklisch zu erregen, **dadurch gekennzeichnet, dass** die besagten Ablaufsteuerungsmittel durch zwei Multiplexer (41, 42) gebildet sind, die in Kaskade zwischen der besagten Ultraschallsignal- Sendestufe und der besagten Sonde (1) angeordnet sind, ein erster Multiplexer (41), der mit ersten Steuermitteln (SY1) ausgestattet ist, um die Wechselfolge zwischen einerseits den für den besagten ersten Transducer (2) bestimmten Signalen und andererseits den für den zweiten und dritten Transducer (3, 4) bestimmten Signalen zu bewirken und ein zweiter Multiplexer (42), der hinter dem besagten ersten Multiplexer angeordnet und mit zweiten Steuermitteln (SY2) ausgestattet ist, um die Wechselfolge zwischen den für den zweiten (3) bzw. den dritten Transducer (4) bestimmten Signalen zu bewirken.

**2.** Ultraschall-Echographiesystem nach Anspruch 1, **dadurch gekennzeichnet, dass** die besagte Signalverarbeitungsstufe Verarbeitungsmittel aufweist, um gesondert und in Echtzeit die drei Ultraschallbilder auf der Anzeigevorrichtung (21) anzuzeigen, die aus den ersten (2), zweiten und dritten Transducern (3, 4) erhalten wurden.

**FIG.1**

**FIG.4**

FIG.2

FIG.3

**FIG.5**